# EUROPEAN PATENT APPLICATION

(11) **EP 1 405 635 A1**
(43) Date of publication of application: **07.04.2004**
(21) Application number: 02730831.1
(22) Date of filing: 31.05.2002
(51) Int. Cl.: A61K 9/20, A61K 47/26, A61K 47/30, A61K 47/32, A61K 47/36, A61K 47/38

(54) **FUNCTIONAL GRAIN-CONTAINING PREPARATIONS QUICKLY DISINTEGRATED IN THE ORAL CAVITY**

(30) Priority: 07.06.2001 JP 2001172528
(71) Applicant: TANABE SEIYAKU CO., LTD., Chuo-ku, Osaka-shi, Osaka 541-8505 (JP)
(72) Inventor: ISHIBASHI, Takashi, Sakai-shi, Osaka 591-8025 (JP); NAGAO, Keigo, Kawanishi-shi, Hyogo 666-0125 (JP); KIYOMIZU, Kosuke, Kyoto-shi, Kyoto 601-8328 (JP)
(74) Representative: Hayes, Adrian Chetwynd
(86) International application number: PCT/JP2002/005355
(87) International publication number: WO 2002/100381

(57) **Abstract**

The present invention provides a process for producing an orally fast disintegrating preparation containing functional particles, which comprises filling in a mold an aqueous dispersion containing (a) a dispersant showing a dispersion maintaining ratio of about 75% or more and a viscosity of about 100 mPa.s or less at 25°C in case of being contained homogeneously in water at 1% by weight, (b) a water-soluble saccharide, and (c) functional particles; and then removing water; and an orally fast disintegrating preparation containing functional particles, which comprises (a) a dispersant showing a dispersion maintaining ratio of 75% or more and a viscosity of 100 mPa.s or less at 25°C in case of being contained homogeneously in water at 1% by weight, (b) a water-soluble saccharide, and (c) functional particles.

## Description

### Technical Field

The present invention relates to an orally fast disintegrating preparation containing functional particles and a process for producing it, and more particularly, to an orally fast disintegrating preparation containing functional particles and a process for producing it, in which the functional particles are not subjected to damage by compression molding, there are no variations in the content of functional particles among preparations, the oral feeling at dosing is satisfactory, the preparation is inert to bacterial growth and a drying step is easy.

### Background Art

Preparations in the form of powders, granules, tablets, capsules and so forth have conventionally been used as solid preparations for oral administration. However, since these forms of preparations present problems such as being difficult to handle as a results of being too small or being difficult to swallow for infants, elderly persons and patients with serious illnesses, various types of preparations that minimize these problems, such as an orally fast disintegrating preparation, are being developed.

On the other hand, preparations have been researched and even used clinically that are imparted with various functions such as controlling the rate of release of drug from the preparation or masking of the taste of the drug contained in the preparation for the purpose of improving therapeutic effect or improving patient compliance.

Therefore, in order to develop a preparation which has such functions while also having a suitable size and improved intakability with respect to easy handling and easier swallowing for infants, elderly persons and patients with serious illnesses, the development of an orally fast disintegrating preparation has been proposed that incorporates particles having the aforementioned functions (functional particles).

Examples of process for producing orally fast disintegrating preparations are described in Japanese Unexamined PCT Publication No. 503237/1995, Japanese Unexamined Patent Publication No. 271054/1993 and Japanese Unexamined Patent Publication No. 291051/1996.

Japanese Unexamined PCT Publication No. 503237/1995 describes that porous tablets containing a drug to be difficult to compress which tablets can be chewed due to softness thereof and can disintegrate rapidly are obtained by compression molding a drug with a meltable binder, melting the binder contained in the resulting preparation and then solidifying.

Japanese Unexamined Patent Publication No. 271054/1993 describes that orally dissolving tablets which have suitable strength and are rapidly soluble and disintegrating in an oral cavity are obtained by forming tablets from a mixture containing a drug, saccharide and water which is contained to a degree that the particle surface of the saccharide is moistened.

Japanese Unexamined Patent Publication No. 291051/1996 describes a process for production of rapidly-dissolving tablets comprising a tablet-forming step in which a tablet material in a dry state containing a drug, water-soluble binder and water-soluble excipient is compression molded at low pressure; a moisturizing step for allowing the tablets molded in said step to absorb moisture; and a drying step in which the moisturized tablets are dried.

However, since each of these processes contains a compression molding step, when orally fast disintegrating preparation containing functional particles are attempted to be produced using these methods, preparations in which the function of the functional particles is maintained cannot be obtained due to the damage on the functional particles by compression.

On the other hand, examples of process for producing preparations that rapidly disintegrate in the oral cavity but do not comprise any compression molding step as described above are described in Japanese Unexamined Patent Publication No. 44619/1978, WO 93/12769, Japanese Unexamined PCT Publication No. 502622/1997 and Japanese Unexamined Patent Publication No. 116466/1999. In these processes, a solution or dispersion containing a drug, water-soluble naturally-occurring polymer substance (e.g., gelatin, agar, xanthane gum, guar gum or dextrin), saccharide and so forth is filled into a casting mold and then dried to produce preparations that rapidly disintegrate in the oral cavity.

However, since preparations obtained by these processes use a naturally-occurring polymer substance, they are susceptible to bacterial growth, and when the polymer substance is dissolved in water, the solution has high viscosity and result in string-like stickiness in the oral cavity, thereby causing an unpleasant feeling at dosing.

Moreover, when preparations containing functional particles are attempted to be produced with these processes, since the functional particles either sink or float in the dispersion during the production process, there is also the problem of obtaining non-uniform preparations in which the content of functional particles varies among the resulting preparations or the distribution of functional particles within the same preparation is unbalanced.

In addition, Japanese Unexamined Patent Publication No. 116464/1999 describes that a rapidly-dissolving solid preparation is obtained by removing the moisture by air-drying and so forth at normal temperatures from a creamy composition in the form of an aqueous suspension obtained by mixing a drug, a saccharide the solubility of which in water is 30 g/100 g or less, and a saccharide the solubility of which in water is 30 g/100 g or more in water. In this process, although neither compression molding nor a naturally-occurring polymer substance is used, in addition to the fact that drying requires a considerable period of time, since the suspension is made to be in the form of a creamy composition having a large stirring resistance, when preparations containing functional particles are attempted to be produced with this process, it is difficult to uniformly disperse the functional particles therein.

### DISCLOSURE OF THE INVENTION

Therefore, in order to solve the problems, the inventors of the present invention conducted extensive research to develop an orally fast disintegrating preparation containing functional particles, and a process for producing it, in which the functional particles are not subjected to damage by compression molding, there are no variations in the content of functional particles among preparations, the oral feeling at dosing is satisfactory, the preparation is inert to bacterial growth and a drying step is easy. Consequently, they have found that when an orally fast disintegrating preparation containing functional particles is produced using an aqueous dispersion containing a dispersant that produces a high dispersion maintaining ratio and low viscosity when contained homogeneously in water, a water-soluble saccharide and functional particles, the aforementioned problems are solved, thereby leading to completion of the present invention.

Namely, the present invention relates to a process for producing an orally fast disintegrating preparation containing functional particles, which comprises filling in a mold an aqueous dispersion containing (a) a dispersant showing a dispersion maintaining ratio of about 75% or more and a viscosity of about 100 mPa^{·}s or less at 25°C in case of being contained homogeneously in water at 1% by weight, (b) a water-soluble saccharide, and (c) functional particles; and then removing water.

In addition, the present invention relates to an orally fast disintegrating preparation containing functional particles, which comprises (a) a dispersant showing a dispersion maintaining ratio of 75% or more and a viscosity of 100 mPa^{·}s or less at 25°C in case of being contained homogeneously in water at 1% by weight, (b) a water-soluble saccharide, and (c) functional particles.

Moreover, the present invention relates to an orally fast disintegrating preparation containing functional particles, which is obtainable by filling in a mold an aqueous dispersion containing (a) a dispersant showing a dispersion maintaining ratio of 75% or more and a viscosity of 100 mPa^{·}s or less at 25°C in case of being contained homogeneously in water at 1% by weight, (b) a water-soluble saccharide, and (c) functional particles; and then removing water.

### BRIEF DESCRIPTION OF THE DRAWINGS

Fig. 1 shows the dissolution characteristics of diltiazem hydrochloride from coated fine particles obtained in Example 4 (1) and from orally fast disintegrating tablets of the present invention obtained in Example 4 (2).
Fig. 2 shows the dissolution characteristics of diltiazem hydrochloride from coated fine particles obtained in Example 6 (1) and from orally fast disintegrating tablets of the present invention obtained in Example 6 (2).
Fig. 3 shows the storage stability of orally fast disintegrating tablets of the present invention obtained in Example 6 (2).
Fig. 4 shows the dissolution characteristics of diltiazem hydrochloride from coated fine particles obtained in Example 7 (1) and from orally fast disintegrating tablets of the present invention obtained in Example 7 (2) and Example 8.
Fig. 5 shows the dissolution characteristics of acetaminophen from coated fine particles obtained in Example 9 (1) and from orally fast disintegrating tablets of the present invention obtained in Example 9 (2) and Example 10.
Fig. 6 shows the dissolution characteristics of ecabet sodium from coated fine particles obtained in Example 17 (1) and from orally fast disintegrating tablets of the present invention obtained in Example 17 (2).
Fig. 7 shows the dissolution characteristics of diltiazem hydrochloride from coated fine particles obtained in Example 18 (1) and from orally fast disintegrating tablets of the present invention obtained in Example 18 (2).
Fig. 8 shows the dissolution characteristics of diltiazem hydrochloride from coated fine particles obtained in Example 18 (1) and from orally fast disintegrating tablets of the present invention obtained in Example 19 (2).

### BEST MODE FOR CARRYING OUT THE INVENTION

The orally fast disintegrating preparation containing functional particles obtained by the process of the present invention has the characteristics indicated below.
1. Since compression molding is not employed in the production process, the function of the functional particles contained therein is maintained without being impaired.
2. A dispersant is used which demonstrates a high dispersion maintaining ratio of about 75% or more at 25°C in the case of being contained homogeneously in water at 1% by weight. Therefore, even when using only a small amount of dispersant, a preparation is obtained
   in which the dispersed state of the functional particles in the aqueous dispersion is satisfactorily maintained during filling in a mold and during removing the water after filling regardless of the specific gravity, water repellency and so forth of the functional particles,
   in which there are no variations in the content of functional particles among preparations, and
   in which the functional particles are uniformly distributed even within the same preparation. Moreover, only a small amount of dispersant is required to be used since a dispersant having a high dispersion maintaining ratio is used.
3. Since a dispersant is used which demonstrates low viscosity of about 100 mPa^{·}s or less at 25°C in the case of being contained homogeneously in water at 1% by weight, the stirring resistance of the aqueous dispersion is so low that it is easy to obtain a homogeneous dispersion, and there is no string-like stickiness in the oral cavity after disintegration, thereby making the oral feeling when taking a preparation produced in this manner satisfactory.
4. Since the resulting preparation does not contain a large amount of naturally-occurring polymer substance, it is inert to bacterial growth.

The functional particles contained in an orally fast disintegrating preparation containing functional particles produced with the process of the present invention refer to any particles, which contain a desired drug and are subjected to measures such as coating, microsphere conversion or matrix formation for the purpose of not only containing a drug, but also controlling the release of the contained drug (slow releasing, gastrosoluble, enterosoluble, colon targeting, etc.), masking the taste of the drug itself, blocking light, retaining moisture and so forth. The particles are subject to no particular restrictions provided that they do not dissolve in the aqueous dispersion during production.

Although there are no particular restrictions on the particle diameter of these functional particles, an average particle diameter of about 45-250 µm is preferable in terms of preventing a rough feeling at dosing after disintegrating in the oral cavity. Functional particles, the average particle diameter of which is 50-200 µm, and in which the ratio of functional particles having a particle diameter of 250 µm or more is 10% or less of the total amount of functional particles, can be used particularly preferably.

Specific examples of the functional particles include coated particles provided with a coating layer surrounding a core particle that contains drug for the purposes previously described, and microspheres or matrix particles in which a drug is included in a water-insoluble substance. The functional particles contained in the orally fast disintegrating preparation containing functional particles according to the process of the present invention and the process for producing them will later be described in detail.

Dispersants that can be used in the process of the present invention are those showing the dispersion maintaining ratio at 25°C is about 75% or more, and the viscosity is about 100 mPa·s or less in the case of being homogeneously contained in water at 1% by weight.

The dispersion maintaining ratio in the process of the present invention refers to the value that expresses to what extent the dispersed state can be maintained as compared with the case of the dispersed state being completely maintained, when nifedipine particles (average particle diameter: 50-100 µm, solubility in water at 25°C: 1 mg/ml or less) are uniformly dispersed in a liquid homogeneously containing dispersant at 1% by weight and the dispersion allowed to stand undisturbed for 5 hours. More specifically, 1 part by weight of nifedipine particles are uniformly dispersed in 99 parts by weight of liquid homogeneously containing dispersant at 1% by weight. 50 ml of the resulting dispersion are poured into a Nessler tube (inner diameter: 22 mm) and allowed to stand undisturbed for 5 hours (25°C). After standing, 1 ml of the dispersion was sampled from the longitudinally central portion of the dispersion followed by measurement of the concentration of nifedipine particles. The ratio of the concentration of particles in the center portion of the dispersion after standing was then taken to be the dispersion maintaining ratio on the basis that the theoretical particle concentration of the dispersion (namely, amount of particles / amount of dispersion) is 100.

The dispersion maintaining ratio in the case of homogeneously containing a dispersant capable of being used in the process of the present invention in water at 1% by weight is about 75% or more, and preferably about 90% or more, under the aforementioned measuring conditions.

In addition, the viscosity (25°C) as measured with a type B viscometer in the case of homogeneously containing a dispersant capable of being used in the process of the present invention in water at 1% by weight is about 100 mPa^{·}s or less, and preferably about 50 mPa^{·}s or less.

In the process of the present invention, any dispersant can be used provided that it is a dispersant having a dispersion maintaining ratio and viscosity as indicated above.

Such dispersants include complexes composed of fine particles of a water-insoluble substance and a water-soluble substance, and particularly complexes obtainable by performing drying treatment on an aqueous dispersion containing fine particles of a water-insoluble substance and a water-soluble substance.

Examples of the water-insoluble substance include fibrous substances that are insoluble in water, and a particular example is microcrystalline cellulose. The fine particles of the water-insoluble substance preferably have an average particle diameter of 30 µm or less, particularly preferably 15 µm or less, and most preferably 10 µm or less.

Examples of the water-soluble substance include locust bean gum, guar gum, tamarind gum, quince seed gum, karaya gum, gum Arabic, tragacanth gum, gaty gum, arabinogalactan, agar, carrageenan, alginic acid and its salts, furcellaran, pectin, marmelo, xanthane gum, cardran, pullulan, dextran, gellan gum, gelatin, cellulose derivatives such as sodium carboxymethyl cellulose, sodium polyacrylate, sodium chondroitin sulfate, water-soluble starch derivatives such as sodium glycolate starch, starch hydrolysates, dextrins, glucose, fructose, xylose, sorbose, sucrose, lactose, maltose, isomerized sugar, coupling sugar, palatinose, neo sugar, reduced starch saccharified syrup, lactulose, polydextrose, fructooligosaccharide, galactooligosaccharide, xylitol, mannitol, maltitol, and sorbitol. Sodium carboxymethyl cellulose, karaya gum, xanthane gum and dextrin are particularly preferable.

Specific examples of the dispersant include microcrystalline cellulose coated with sodium carboxymethyl cellulose (e.g., Avicel RC591NF and Avicel CL611, both available from Asahi Kasei), microcrystalline cellulose coated with karaya gum and dextrin (e.g., Avicel RC-N81, available from Asahi Kasei), microcrystalline cellulose coated with xanthane gum and dextrin (e.g., Avicel RC-N30, available from Asahi Kasei), and compositions comprising microcrystalline cellulose and a water-soluble substance described in Japanese Unexamined Patent Publication No. 70365/1995, Japanese Unexamined Patent Publication No. 102113/1995, Japanese Unexamined Patent Publication No. 173332/1995, Japanese Unexamined Patent Publication No. 268129/1995, Japanese Unexamined Patent Publication No. 151481/1996 and Japanese Unexamined Patent Publication No. 3243/1997. Microcrystalline cellulose coated with sodium carboxymethyl cellulose is most preferable.

In the process of the present invention, although water-soluble saccharides are also contained in the aqueous dispersion in addition to the aforementioned dispersant and functional particles, due to saccharides contained therein, the resulting preparation not only has the required hardness, but also is able to be rapidly disintegrated in the oral cavity. Moreover, said preparation has the effect of preventing the occurrence of string-like stickiness following disintegration in the oral cavity.

Saccharide the solubility of which in water at 25°C is 5% or more can be used for the water-soluble saccharide in the process of the present invention, and these water-soluble saccharides may be used alone or in combination. More specifically, one or more than one selected from glucose, fructose, sucrose, lactose, maltose, mannitol, xylitol, sorbitol, trehalose and erythritol can be used, and one or more than one selected from lactose, maltose, mannitol and erythritol can be used particularly preferably. Preferable combinations in the case of combining two or more saccharides are the combination of lactose and mannitol, the combination of mannitol and erythritol, the combination of lactose and erythritol and the combination of mannitol and maltose.

Desired additives can also be used in the process of the present invention in addition to the aforementioned functional particles, dispersants and water-soluble saccharides. There are no particular restrictions on these additives and any additive may be used, provided that it does not have a detrimental effect on the intakability, hardness or disintegration time of the preparation produced. Specific examples of the additive include sweeteners such as aspartame, sodium saccharin, saccharin, stevia, glycyrrhizin and potassium acesulfame; acidulants such as citric acid, tartaric acid and malic acid; flavoring such as menthol, peppermint oil, peppermint, orange, lemon-lime, lemon and strawberry; and colorants such as caramel, annatto extract pigment, β-carotene and beet red.

In the production of an orally fast disintegrating preparation containing functional particles according to the process of the present invention, an aqueous dispersion is first prepared by adding functional particles, water-soluble saccharide and dispersant to water. The blending ratios and blended amounts of the functional particles, water-soluble saccharide and dispersant blended can be suitably determined according to the type of drug contained in the functional particles, application of the orally fast disintegrating preparation containing functional particles to be produced and so forth. However, the content of the dispersant in the aqueous dispersion relative to the total weight of the aqueous dispersion is preferably 0.05-2.0% by weight, and more preferably 0.1-0.8% by weight. The blended amount of the water-soluble saccharide is 30-1000 parts by weight, preferably 30-500 parts by weight, and more preferably about 50-400 parts by weight, relative to 1 part by weight of the dispersant, and the blended amount of the functional particles is 1-500 parts by weight, and preferably about 10-200 parts by weight, relative to 1 part by weight of the dispersant. There are no particular restrictions on the order of addition provided that a homogeneous aqueous dispersion is ultimately obtained. Preferably, functional particles and water-soluble saccharide are added after dispersing the dispersant in the water. Addition to water is preferably carried out while stirring. Examples of stirring method that can be used include a magnetic stirrer, propeller stirring, a homomixer and a homogenizer. Although the water-soluble saccharide preferably does not completely dissolve in the aqueous dispersion, it may be completely dissolved.

Next, the prepared aqueous dispersion is filled in a mold. Any mold can be used provided that it has the function of a mold, and examples of the mold that can be used include those made of metal, resin film and so forth. A resin film sheet having a large number of wells for housing tablets which may be used for PTP packaging can be used preferably. In the case of using as the mold the above-mentioned resin film sheet for PTP packaging having a large number of wells for housing tablets, the product form can be obtained directly by removing moisture by drying and so forth after filling with the aqueous dispersion, and then attaching a cover sheet for PTP packaging. Polypropylene, polyvinyl chloride or polyvinylidene chloride sheets can be used for the resin film sheet. There are no particular restrictions on the shape of the mold, examples of which include that having a shape of the desired size, such as that containing cylindrical wells which have a diameter of 5-20 mm and a height of 2-10 mm. The aqueous dispersion prepared in the previously described manner is then filled in this mold followed by removal of water from the aqueous dispersion. Any method may be used to remove the water provided that it does not have an effect on any of the components contained in the aqueous dispersion. Examples of such method include drying methods, such as air drying, blow drying, vacuum drying and freeze drying, with freeze drying being preferable. Freeze drying should be carried out in accordance with ordinary methods by freezing the aqueous dispersion filled in the mold and then sublimating the moisture by placing it under reduced pressure, and this can be carried out easily using known freeze drying equipment.

An orally fast disintegrating preparation containing functional particles of the present invention that has been prepared according to the aforementioned method contains: (a) a dispersant showing the dispersion maintaining ratio of about 75% or more and the viscosity of about 100 mPa^{·}s or less at 25°C in the case of being homogeneously contained in water at 1% by weight, (b) a water-soluble saccharide, and (c) the aforementioned functional particles. In the present preparation, the content of the dispersant is about 0.07-4% by weight, preferably about 0.07-2.8% by weight, and more preferably about 0.14-1.12% by weight. The blended amount of the water-soluble saccharide is 30-1000 parts by weight, preferably 30-500 parts by weight, and more preferably about 50-400 parts by weight, relative to 1 part by weight of the dispersant, while the blended amount of the functional particles is 1-500 parts by weight, and preferably about 10-200 parts by weight, relative to 1 part by weight of the dispersant.

In addition, the hardness of the present preparation is 10 N or more, preferably about 19.6 N or more, and more preferably about 29.4 N or more (in the case of measuring hardness with a Tablet Tester (Model 6D, manufactured by Freund Industrial Co., Ltd.). In addition, the disintegration time (test solution: water) of the present preparation in accordance with the disintegration test method contained in the 13th Revised Edition of the Japanese Pharmacopoeia is preferably about 60 seconds or shorter and more preferably about 30 seconds or shorter, while the disintegration time in the oral cavity is preferably about 60 seconds or shorter, more preferably about 30 seconds or shorter, and most preferably about 20 seconds or shorter.

Although the functional particles contained in the orally fast disintegrating preparation containing functional particles in the process of the present invention are defined as previously described, the following provides a detailed explanation of these functional particles. The functional particles may contain any drug provided that it is a drug that can be administered orally, and there are no particular restrictions on its type. The following provides a list of examples of drug that can be contained.
(1) Antipyretics, analgesics and antiphlogistics (such as indometacin, acetylsalicylic acid, diclofenac sodium, ketoprofen, ibuprofen, mefenamic acid, azulene, phenacetin, isopropyl antipyrine, acetaminophen, benzadac, phenylbutazone, flufenamic acid, sodium salicylate, salicylamide, sasapyrine and etodolac);
(2) Steroidal anti-inflammatory drugs (such as dexamethasone, hydrocortisone, predisolone and triamcinolone);
(3) Anti-ulcer drugs (such as ecabet sodium, enprostil, sulpiride, cetraxate hydrochloride, gefarnate, irsogladine maleate, cimetidine, ranitidine hydrochloride, famotidine, nizatidine, roxatidine acetate hydrochloride, omeprazole and lansoprazole);
(4) Coronary vasodilators (such as nifedipine, isosorbide nitrate, diltiazem hydrochloride, trapidil, dipyridamole, dilazep hydrochloride, verapamil, nicardipine, nicardipine hydrochloride and verapamil hydrochloride);
(5) Peripheral vasodilators (such as ifenprodil tartrate, cinepazide maleate, cyclandelate, cinnarizine and pentoxyfylline);
(6) Antibiotics (such as ampicillin, amoxicillin, cefalexin, erythromycin ethyl succinate, bacampicillin hydrochloride, minocycline hydrochloride, chloramphenicol, tetracycline, erythromycin, ceftazidime, cefuroxime sodium, aspoxicillin and ritipenemacoxil hydrate);
(7) Synthetic antimicrobials (such as nalidixic acid, piromidic acid, pipemidic acid trihydrate, enoxacin, cinoxacin, ofloxacin, norfloxacin, ciprofloxacin hydrochloride and sulfamethoxazole·trimethoprim);
(8) Antiviral agents (such as aciclovir and ganciclovir);
(9) Antipasmodics (such as propantheline bromide, atropine sulfate, oxapium bromide, timepidium bromide, scopolamine butylbromide, trospium chloride, butropium bromide, N-methylscopolamine methylsulfate and methyloctatropine bromide);
(10) Antitussives (such as tipepidine hibenzate, methylephedrine hydrochloride, codeine phosphate, tranilast, dextromethorphan hydrobromide, dimemorfan phosphate, clobutinol hydrochloride, fominoben hydrochloride, benproperine phosphate, eprazinone hydrochloride, clofedanol hydrochloride, ephedrine hydrochloride, noscapine, pentoxyverine citrate, oxeladin citrate and isoaminil citrate);
(11) Expectorants (such as bromhexine hydrochloride, carbocisteine, ethyl cysteine hydrochloride and methylcysteine hydrochloride);
(12) Bronchodilators (such as theophylline, aminophylline, sodium cromoglicate, procaterol hydrochloride, trimetoquinol hydrochloride, diprophylline, salbutamol sulfate, clorprenaline hydrochloride, formoterol fumarate, orciprenaline sulfate, pilbuterol hydrochloride, hexoprenaline sulfate, bitolterol mesilate, clenbuterol hydrochloride, terbutaline sulfate, mabuterol hydrochloride, fenoterol hydrobromide and methoxyphenamine hydrochloride);
(13) Cardiacs (such as dopamine hydrochloride, dobutamine hydrochloride, docarpamine, denopamine, caffeine, digoxin, digitoxin and ubidecarenone);
(14) Diuretics (such as furosemide, acetazolamide, trichlormethiazide, methyclothiazide, hydrochlorothiazide, hydroflumethazide, etiazide, cyclopenthiazide, spironolactone, triamterene, florothiazide, piretanide, mefruside, etacrynic acid, azosemide and clofenamide);
(15) Muscle relaxants (such as chlorphenesin carbamate, tolperisone hydrochloride, eperisone hydrochloride, tizanidine hydrochloride, mephenesin, chlorzoxazone, phenprobamate, methocarbamol, chlormezanone, pridinol mesilate, afloqualone, baclofen and dantrolene sodium);
(16) Cerebral metabolism activator (such as nicergoline, meclofenoxate hydrochloride and taltirelin);
(17) Minor tranquilizers (such as oxazolam, diazepam, clotiazepam, medazepam, temazepam, fludiazepam, meprobamate, nitrazepam and chlordiazepoxide);
(18) Major tranquilizers (such as sulpiride, clocapramine hydrochloride, zotepine, chlorpromazine and haloperidol);
(19) β-blockers (such as bisoprolol fumarate, pindolol, propranolol hydrochloride, carteolol hydrochloride, metoprolol tartrate, labetalol hydrochloride, acebutolol hydrochloride, bufetolol hydrochloride, alprenolol hydrochloride, arotinolol hydrochloride, oxprenolol hydrochloride, nadolol, bucumolol hydrochloride, indenolol hydrochloride, timolol maleate, befunolol hydrochloride and bupranolol hydrochloride);
(20) Antiarrhythmics (such as procainamide hydrochloride, disopyramide, ajmaline, quinidine sulfate, aprindine hydrochloride, propafenone hydrochloride, mexiletine hydrochloride and azimilide hydrochloride);
(21) Antipodagrics (such as allopurinol, probenecid, colchicine, sulfinpyrazone, benzbromarone and bucolome);
(22) Anticoagulants (such as ticlopidine hydrochloride, dicoumarol, warfarin potassium and (2R,3R)-3-acetoxy-5-[2-(dimethylamino)ethyl]-2,3-dihydro-8-methyl-2-(4-methylphenyl)-1,5-benzothiazepin-4(5H)-one maleate);
(23) Thrombolytic drugs (such as methyl(2E,3Z)-3-benzylidene-4-(3,5-dimethoxy-α-methylbenzylidene)-N-(4-methylpiperazin-1-yl)succinate hydrochloride);
(24) Hepatotonics (such as (±) r-5-hydroxymethyl-t-7-(3,4-dimethoxyphenyl)-4-oxo-4,5,6,7-tetrahydrobenzo[b]-furan-c-6-carboxylic acid lactone);
(25) Antiepileptics (such as phenytoin, valproate sodium, metarpital and carbamazepine);
(26) Antihistamines (such as chlorpheniramine maleate, clemastine fumarate, mequitazine, alimemazine tartrate, cyproheptadine hydrochloride and bepotastine besilate);
(27) Antiemetics (such as difenidol hydrochloride, metoclopramide, domperidone, betahistine mesilate and trimebutine maleate);
(28) Hypotensive drugs (such as dimethylaminoethyl reserpilinate hydrochloride, rescinnamine, methyldopa, prazosin hydrochloride, bunazocin hydrochloride, clonidine hydrochloride, budralazine, urapidil and N-[6-[2-[(5-bromo-2-pyrimidinyl)oxy]ethoxy]-5-(4-methylphenyl)-4-pyrimidinyl]-4-(2-hydroxy-1,1-dimethylethyl) benzenesulfonamide sodium);
(29) Hypolipidemic drugs (such as pravastatin sodium and fluvastatin sodium);
(30) Sympathomimetic agents (such as dihydroergotamine mesilate, isoproterenol hydrochloride and etilefrine hydrochloride);
(31) Oral antidiabetics (such as glibenclamide, tolbutamide and glimidine sodium);
(32) Oral carcinostatic agents (such as marimastat);
(33) Alkaloid narcotics (such as morphine, codeine and cocaine);
(34) Vitamins (such as vitamin B1, vitamin B2, vitamin B6, vitamin B12, vitamin C and folic acid);
(35) Drugs for treatment of pollakiuria (such as flavoxate hydrochloride, oxybutynin hydrochloride and terolidine hydrochloride);
(36) Angiotensin converting enzyme inhibitors (such as imidapril hydrochloride, enalapril maleate, alacepril and delapril hydrochloride).

As has been described above, specific examples of functional particles include coated particles provided with a coating layer around a core particle that contains a drug, for the purpose of controlling the release of the drug contained therein, masking the taste of the drug, blocking light or retaining moisture and so forth; and microspheres or matrix particles in which a drug is included in a water-insoluble substance. Any component can be used for the component contained in a coating layer in the case of using coated particles as the functional particles, provided that it fulfills a function such as controlling the release of the drug contained in the coated particles, masking its taste, blocking light and retaining moisture. Specific examples of coating agent that can be used include coating agents such as water-soluble polymer, water-insoluble polymer, enterosoluble polymer, gastrosoluble polymer and hydrophobic organic compound.

Examples of the water-soluble polymer include (1) water-soluble cellulose ethers such as methyl cellulose, hydroxypropyl cellulose and hydroxypropyl methyl cellulose; (2) water-soluble polyvinyl derivatives such as polyvinyl pyrrolidone and polyvinyl alcohol; and (3) alkylene oxide polymers such as polyethylene glycol and polypropylene glycol.

Examples of the water-insoluble polymer include (1) water-insoluble cellulose ethers such as ethyl cellulose; and (2) water-insoluble acrylic acid copolymers such as ethyl acrylate-methyl methacrylate-methacryloyloxyethyl trimethyl ammonium chloride ethyl copolymer (e.g., trade name: Eudragit RS, available from Roehm-Pharma) and methyl methacrylate-ethyl acrylate copolymer (e.g., trade name: Eudragit NE30D, available from Roehm-Pharma).

Examples of the enterosoluble polymer include (1) enterosoluble cellulose derivatives such as hydroxypropylmethyl cellulose acetate succinate, hydroxypropylmethyl cellulose phthalate, hydroxymethylethyl cellulose phthalate, cellulose acetate phthalate, cellulose acetate succinate, cellulose acetate maleate, cellulose benzoate phthalate, cellulose propionate phthalate, methyl cellulose phthalate, carboxymethylethyl cellulose and ethylhydroxyethyl cellulose phthalate; (2) enterosoluble acrylic acid copolymers such as styrene-acrylic acid copolymer, methyl acrylate-acrylic acid copolymer, methyl acrylate-methacrylic acid copolymer, butyl acrylate-styrene-acrylic acid copolymer, methacrylic acid-methyl methacrylate copolymer (e.g., trade name: Eudragit L100, Eudragit S, both available from Roehm-Pharma), methacrylic acid-ethyl acrylate (e.g., trade name: Eudragit L100-55, available from Roehm-Pharma), and methyl acrylate-methacrylic acid-octyl acrylate copolymer; (3) enterosoluble maleic acid copolymers such as vinyl acetate-maleic anhydride copolymer, styrene-maleic anhydride copolymer, styrene-maleic acid monoester copolymer, vinylmethyl ether-maleic anhydride copolymer, ethylene-maleic anhydride copolymer, vinylbutyl ether-maleic anhydride copolymer, acrylonitrile-methyl acrylate-maleic anhydride copolymer, and butyl acrylate-styrene-maleic anhydride copolymer; and (4) enterosoluble polyvinyl derivatives such as polyvinyl alcohol phthalate, polyvinyl acetal phthalate, polyvinyl butyrate phthalate and polyvinyl acetoacetal phthalate.

Examples of the gastrosoluble polymer include (1) gastrosoluble polyvinyl derivatives such as polyvinyl acetal diethylaminoacetate; and (2) gastrosoluble acrylic acid copolymers such as methyl methacrylate-butyl methacrylate-dimethyl aminoethyl methacrylate copolymers (e.g., trade name: Eudragit E, available from Roehm-Pharma).

Examples of the hydrophobic compound include (1) higher fatty acids such as stearic acid, lauric acid, myristic acid, palmitic acid and behenic acid; (2) higher alcohols such as lauryl alcohol, myristyl alcohol, cetyl alcohol, stearyl alcohol and behenyl alcohol; (3) triglycerides of higher fatty acids such as stearic triglyceride, myristic triglyceride, palmitic triglyceride and lauric triglyceride; and (4) hydrogenated and non-hydrogenated natural oils and fats such as hydrogenated castor oil, hydrogenated coconut oil and beef tallow.

In addition to the coating agent components like those described above, colorant, masking agent, plasticizer, lubricant and various other additives can be additionally contained in this coating layer if necessary. Examples of the colorant include edible pigment, lake pigment, caramel, carotene, annatto, cochineal and iron sesquioxide, as well as the opaque colorant Opalux consisting primarily of lake pigment and syrup. Specific examples of the colorant that can be used include edible aluminum lake pigments, such as edible red dyes no. 2 and no. 3, edible yellow dyes no. 4 and no. 5, edible green dye no. 3, edible blue dyes no. 1 and no. 2, and edible purple dye no. 1; annatto (natural pigment originating in Bixa orellana); carmine (aluminum carminate); and pearl essence (consisting primarily of guanine). Examples of the masking agent that can be used include titanium dioxide, precipitated calcium carbonate, calcium hydrogen phosphate and calcium sulfate. Examples of the plasticizer that can be used include phthalic acid derivatives such as diethyl phthalate, dibutyl phthalate and butylphthalylbutyl glycolate; silicon oil; triethyl citrate; triethyl acetylcitrate; triacetin; propylene glycol and polyethylene glycol. In addition, examples of the lubricant that can be used include magnesium stearate, talc, synthetic magnesium silicate and fine granular silicon oxide.

Specific examples of the coating layer which the coated particles have include a mixed coating of an enterosoluble polymer and hydrophobic organic compound (Japanese Unexamined Patent Publication No. 2000-103732), and a mixed coating of a water-insoluble polymer and hydrophobic organic compound (Japanese Unexamined Patent Publication No. 2000-198747).

In addition, the aforementioned coating layer may comprise one layer or two or more layers. An example of such a coating layer is a multi-layered coating layer, in which all of the adjacent layers contain a mutually different mixture of a hydrophobic organic compound and a water-soluble polymer (Japanese Patent Application No. 2001-018807).

Drug-containing core particles that are coated in coated particles are particles that contain a drug desired to be blended alone or in combination with various pharmaceutical additives ordinarily used in this field, and such particles can be used that have an average particle diameter of preferably about 40-245 µm, and particularly preferably about 45-195 µm.

In addition to a drug, pharmaceutical additives that can be used in preparations for oral administration can be contained in the drug-containing core particles, examples of which include excipient, disintegrating agent, binder, lubricant, surfactant, flavoring, colorant, sweetener and solubilizing aid. Examples of the excipient that can be used include lactose, saccharose, mannitol, xylitol, erythritol, sorbitol, maltitol, calcium citrate, calcium phosphate, crystalline cellulose and magnesium aluminate metasilicate. Examples of the disintegrating agent that can be used include cornstarch, potato starch, sodium carboxymethyl starch, partial α starch, calcium carboxymethyl cellulose, carboxymethyl cellulose, low-substituted hydroxypropyl cellulose, crosslinked sodium carboxymethyl cellulose and crosslinked polyvinyl pyrrolidone. Examples of the binder that can be used include hydroxypropyl cellulose, hydroxypropylmethyl cellulose, polyvinyl pyrrolidone, polyethylene glycol, dextrin and α starch. Examples of the lubricant that can be used include magnesium stearate, calcium stearate, talc, light silicic anhydride and hydrated silicon dioxide.

In addition, examples of the surfactant that can be used include phospholipids, glycerin fatty acid esters, sorbitan fatty acid esters, polyoxyethylene fatty acid esters, polyethylene glycol fatty acid esters, polyoxyethylene hydrogenated castor oil, polyoxyethylene alkyl ethers and sucrose fatty acid esters. Examples of the flavoring that can be used include orange oil, fennel oil, cinnamon oil, clove oil, turpentine oil, peppermint oil and eucalyptus oil. Examples of the colorant that can be used include edible red dyes no. 2 and no. 3, edible yellow dyes no. 4 and no. 5, edible green dye no. 3, edible blue dyes no. 1 and no. 2, and aluminum lake pigments thereof; iron sesquioxide; and yellow iron sesquioxide. Examples of the sweetener that can be used include saccharin and aspartame. Examples of the solubilizing aid that can be used include cyclodextrin, arginine, lysine and tris-aminomethane.

Drug-containing core particles can be prepared by known granulation methods such as wet granulation, dry granulation, layering granulation, heated granulation, impregnation granulation and spray drying granulation, using the aforementioned drugs and various types of additives as necessary.

The following method can be used to prepare drug-containing core particles using a wet granulation method.
(1) A binder solution is added to a mixture of a drug and various types of pharmaceutical additives (to be referred to as a drug mixture) followed by stirring and granulation using a low shear mixing granulator or high shear mixing granulator.
(2) After adding a binder solution to a drug mixture and kneading, the mixture is granulated and sized using an extruding granulator.
(3) A binder solution is sprayed onto a drug mixture and granulated under fluidization, using a fluidized bed granulator or a rolling stirring fluidized bed granulator.

In preparing drug-containing core particles using a dry granulation method, the drug mixture is granulated using a roller compactor, a roll granulator or the like.

In preparing drug-containing core particles using a layering granulation method, the drug mixture is added while spraying a binder solution onto a rolling inert carrier using a centrifugal fluidized granulator to adhere the drug mixture onto the carrier. Examples of the inert carrier that can be used include crystals of saccharides or inorganic salts such as crystalline lactose, crystalline cellulose and crystalline sodium chloride; and spherical granules [e.g., spherical granules of crystalline cellulose (trade name: Avicel SP, available from Asahi Kasei), spherical granules of crystalline cellulose and lactose (trade name: Nonpareil NP-5, Nonpareil NP-7, available from Freund Industrial Co.), spherical granules of purified saccharose (trade name: Nonpareil 103, available from Freund Industrial Co.) and spherical granules of lactose and α starch].

The following method can be used to prepare drug-containing core particles using a heated granulation method.
(1) A drug mixture containing a substance that is melted by heating (heat-melting substance), such as polyethylene glycol, oil or wax, is granulated by stirring at a temperature at which the heat-melting substance melts using a low shear mixing granulator or high shear mixing granulator.
(2) A drug mixture containing a heat-melting substance is added to an inert carrier rolling at a temperature at which the heat-melting substance melts using a centrifugal fluidized granulator to adhere the drug mixture onto the carrier.

In preparing drug-containing core particles using an impregnation granulation method, a solution containing a drug at a suitable concentration is mixed with a porous carrier, and after the drug solution is adequately retained in the pores of the carrier, the carrier is dried to remove the solvent. Examples of porous carrier that can be used include magnesium aluminate metasilicate (trade name: Neusilin, available from Fuji Chemical Industry) and calcium silicate (trade name: Fluorite, available from Eisai).

In preparing drug-containing core particles using a spray drying granulation method, a drug solution or suspension is sprayed in a high-temperature air flow using a spray dryer or other spray drying device followed by drying.

In addition, in preparing, for example, fine particles having an average particle diameter of about 45-195 µm, wet granulation using a high shear rotary granulator (for example, the method described in Japanese Unexamined Patent Publication No. 2000-128774 in which a binder solution is added to a excipient having the property of retaining a drug and a solvent followed by high-speed rolling granulation), impregnation granulation and spray drying granulation are prefered.

In preparing coated particles as an example of functional particles, the aforementioned coating layer is provided on drug-containing core particles prepared in the manner described above. Any coating method ordinarily used in the field of pharmaceutics technology can be used for providing a coating layer on drug-containing core particles. For example, a coating solution can be prepared by dissolving or dispersing in a solvent a coating agent such as a water-soluble polymer, water-insoluble polymer, enterosoluble polymer, gastrosoluble polymer or hydrophobic organic compound, along with colorant, masking agent, plasticizer or lubricant as necessary, followed by spraying this onto drug-containing core particles using an ordinarily used coating device and by drying to obtain coated particles.

Examples of the solvent that can be used in the coating solution include alcohols such as methanol, ethanol, n-propanol, isopropanol, n-butanol, 2-methoxyethanol (trade name: Methyl Cellosolve, available from Katayama Chemical) and 2-ethoxyethanol (trade name: Cellosolve, available from Katayama Chemical); hydrocarbons such as hexane, cyclohexane, petroleum ether, petroleum benzene, ligroin, benzene, toluene and xylene; ketones such as acetone and methyl ethyl ketone; halogenated hydrocarbons such as dichloromethane, chloroform, carbon tetrachloride, ethylene dichloride, trichloroethylene and 1,1,1-trichloroethane; esters such as methyl acetate, ethyl acetate and butyl acetate; ethers such as isopropyl ether and dioxane; and water. In addition, examples of the coating device that can be used include fluidized bed coating devices, centrifugal fluidized bed coating devices and pan coating devices.

In the case of using microspheres or matrix particles as the functional particles, these functional particles can be easily prepared using known process for producing microsphere and matrix particle, examples of which include, but are not limited to the following methods.
(1) A method in which powder comprising a drug and a water-insoluble substance is adhered around inert core particles while spraying an aqueous binder solution using a centrifugal flow granulation, followed by drying at high temperature.
(2) A method in which a drug and a water-insoluble substance are heated above the melting point of the water-insoluble substance to melt followed by spraying and cooling.
(3) A method in which a drug and a water-insoluble substance are heated above the melting point of the water-insoluble substance to melt followed by dropping at a fixed rate onto a disk rotating at high speed (spray chilling).

Examples of the water-insoluble substance that can be used include saturated fatty acids having 14-22 carbons (e.g., myristic acid, stearic acid, palmitic acid and behenic acid), higher alcohols having 16-22 carbons (e.g., cetyl alcohol and stearyl alcohol), higher alkane mixtures (paraffin) and esters of fatty acids and water-insoluble higher monovalent or divalent alcohols (wax). Examples of the wax that can be used include both vegetable waxes (e.g., hydrogenated cottonseed oil, hydrogenated soybean oil, hydrogenated castor oil and carnauba wax) and animal waxes (e.g., beeswax, spermaceti and lanolin).

Examples of the inert core particles that can be used in the method (1) include those that are similar to inert carriers used in layering granulation, while examples of the binder that can be used include conventional water-soluble binders such as hydroxypropyl cellulose, polyethylene glycol, hydroxypropylmethyl cellulose and polyvinyl pyrrolidone.

Drying at high temperature results in the formation of a matrix caused by melting of the water-insoluble substance due to heating it near its melting point. In this method, a particle diameter of the microspheres or matrix particles formed can be regulated by adjusting the particle diameter of the inert core particles and the adhered amounts of a drug and a water-insoluble substance.

The particle diameter of the microspheres or matrix particles formed can be regulated by adjusting the spray nozzle in the method (2), or by adjusting the disk rotating speed and dripping rate in the method (3).

In addition, the rate of dissolution of a drug from the microspheres or matrix particles formed can be increased by adding a water-soluble low molecular substance, surfactant or disintegrating agent and so forth during production in any of the methods (1) ∼ (3), while conversely, the rate of dissolution of drug from the microspheres or matrix particles formed can be decreased by adding a hydrophobic substance, water-swelling substance or substance that gelates in the presence of water during production.

### Examples

The following examples provide a detailed explanation of orally fast disintegrating preparations containing functional particles and a process for producing them according to the present invention.

### Example 1

(1) 66 parts by weight of ascorbic acid pulverized with a hammer mill (Egg Sample Mill KII GWH-1, manufactured by Fuji Paudal) and 33 parts by weight of microcrystalline cellulose (Avicel PH-M25, available from Asahi Kasei) were stirred (700 rpm) using a high shear mixing granulator (New-Gra Machine NG-200, manufactured by Seishin Enterprises). 400 ml of a hydroxypropyl cellulose solution (prepared by dissolving 1 part by weight of hydroxypropyl cellulose (HPC-SL, available from Nippon Soda) in a mixture of 59.4 parts by weight of ethanol and 39.6 parts by weight of water) were added thereto. The mixture was granulated for 25 minutes with stirring. After drying the resulting particles for 3 hours at 45°C, they were sieved to obtain a fraction having an average particle diameter of 75-150 µm as ascorbic acid-containing core particles.
   A coating solution (prepared by dissolving 20 g of ethyl cellulose (Ethocel #10, available from Dow Chemical), 20 g of methacrylic acid-methyl methacrylate copolymer (Eudragit L100, available from Roehm-Pharma) and 40 g of stearic acid in 920 g of ethanol) was sprayed onto 80 g of the ascorbic acid-containing core particles under fluidization in a Wurster fluidized bed coating device (GPCG-1, manufactured by Glatt) so that the coating ratio (ratio of coating layer to core particles) was 30% by weight to obtain coated fine particles.
(2) 0.16 g of aspartame, 40 g of the ascorbic acid-containing coated fine particles in (1) above, 33.9 g of mannitol and 70.9 g of erythritol were added to 55 g of water containing 0.5% by weight of Avicel RC-591NF (microcrystalline cellulose coated with sodium carboxymethyl cellulose, available from Asahi Kasei) to obtain a coated fine particle dispersion. 500 mg of this coated fine particle dispersion were filled into the holes of a concave mold having a diameter of 10 mm and freeze-dried (conditions: pre-cooling for 3 hours at -40°C and drying for 2 hours at -20°C and 0.1 torr and for 16 hours at 20°C and 0.1 torr) to obtain orally fast disintegrating tablets of the present invention.

When the resulting orally fast disintegrating tablets were administered to three healthy adult males and the disintegration time in the oral cavity was measured, it was found to be 21 seconds on average. This demonstrates superior rapid disintegration in the oral cavity.

### Example 2

930 mg of the coated fine particle dispersion obtained in Example 1 (2) were filled into the holes of a concave mold having a diameter of 15 mm followed by freeze-drying (conditions: same as Example 1(2)) to obtain orally fast disintegrating tablets of the present invention.

When the hardness of the resulting orally fast disintegrating tablets (6 tablets) was measured using a Tablet Tester (Model 6D, manufactured by Freund Industrial Co), it was found to be 38.2 N on average.

In addition, when a disintegration test (test solution: water) was carried out in compliance with the disintegration test method described in the 13th Revised Edition of the Japanese Pharmacopoeia and the disintegration time (3 tablets) was measured, it was found to be 24.3 seconds on average.

On the basis of the above, preparations obtained according to the process of the present invention were confirmed to have high hardness despite the extremely short disintegration time.

### Example 3

0.16 g of aspartame, 1.6 g of lemon flavor, 40 g of the ascorbic acid-containing coated fine particles prepared in Example 1(1), 33.9 g of mannitol and 70.9 g of erythritol were added to 53.4 g of water containing 0.5% by weight of Avicel RC-591NF to obtain a coated fine particle dispersion. 500 mg of this coated fine particle dispersion were filled into the holes of a concave mold having a diameter of 10 mm followed by freeze-drying (conditions: same as Example 1(2)) to obtain orally fast disintegrating tablets of the present invention.

### Example 4

(1) 10 parts by weight of diltiazem hydrochloride pulverized with Sample Mill (manufactured by Fuji Paudal), 69 parts by weight of mannitol and 20 parts by weight of microcrystalline cellulose (Avicel PH-M25, available from Asahi Kasei) were stirred (700 rpm) using a high shear mixing granulator (New-Gra Machine NG-200, manufactured by Seishin Enterprises). 400 ml of a hydroxypropyl cellulose solution (prepared by dissolving 1 part by weight of hydroxypropyl cellulose (HPC-SL, available from Nippon Soda) in a mixture of 59.4 parts by weight of ethanol and 39.6 parts by weight of water) were added thereto. The mixture was granulated for 25 minutes with stirring. After drying the resulting granulated particles for 3 hours at 45°C, they were sieved to obtain a fraction having a particle diameter of 75-150 µm as drug-containing core particles.
   A coating solution (prepared by dissolving 40 g of ethyl cellulose (Ethocel #10, available from Dow Chemical) and 40 g of stearic acid in 920 g of ethanol) was sprayed onto 80 g of the diltiazem hydrochloride-containing core particles under fluidization in a Wurster fluidized bed coating device (GPCG-1, manufactured by Glatt) so that the coating ratio (ratio of coating layer to core particles) was 25% by weight to obtain coated fine particles.
(2) 0.08 g of aspartame, 20 g of the diltiazem hydrochloride-containing coated fine particles in (1) above, 17 g of mannitol and 35.4 g of erythritol were added to 27.5 g of water containing 0.5% by weight of Avicel RC-591NF to obtain a coated fine particle dispersion. 500 mg of this coated fine particle dispersion were filled into the holes of a concave mold having a diameter of 10 mm and freeze-dried (conditions: same as Example 1(2)) to obtain orally fast disintegrating tablets of the present invention.

### Example 5

0.08 g of aspartame, 20 g of the diltiazem hydrochloride-containing coated fine particles prepared in Example 4(1), 17 g of mannitol and 35.4 g of erythritol were added to 27.5 g of water containing 1% by weight of Avicel RC-591NF to obtain a coated fine particle dispersion. 500 mg of this coated fine particle dispersion were filled into the holes of a concave mold having a diameter of 10 mm followed by freeze-drying (conditions: same as Example 1(2)) to obtain orally fast disintegrating tablets of the present invention.

### Example 6

(1) 53.2 parts by weight of diltiazem hydrochloride pulverized with Sample Mill (manufactured by Fuji Paudal), 26.6 parts by weight of mannitol and 19.9 parts by weight of microcrystalline cellulose (Avicel PH-M25, available from Asahi Kasei) were stirred (450 rpm) using a high shear mixing granulator (New-Gra Machine NG-350, manufactured by Seishin Enterprises). 450 ml of a hydroxypropyl cellulose solution (prepared by dissolving 2 parts by weight of hydroxypropyl cellulose (HPC-SL, available from Nippon Soda) in a mixture of 68 parts by weight of ethanol and 30 parts by weight of water) were added thereto. The mixture was granulated for 30 minutes with stirring. After drying the resulting granulated particles for 16 hours at 45°C, they were sieved to obtain a fraction having a particle diameter of 75-150 µm as drug-containing core particles.
   A coating solution (prepared by dissolving 40 g of ethyl cellulose (Ethocel #10, available from Dow Chemical) and 10 g of stearic acid (available from Kao Corp.) in 950 g of ethanol) was sprayed onto 80 g of the diltiazem hydrochloride-containing core particles under fluidization in a Wurster fluidized bed coating device (GPCG-1, manufactured by Glatt) so that the coating ratio (ratio of coating layer to core particles) was 50% by weight to obtain coated fine particles. The resulting coated fine particles were sieved to obtain a fraction having a particle diameter of 75-250 µm as diltiazem hydrochloride-containing coated particles.
(2) 1.2 g of aspartame, 23.4 g of the diltiazem hydrochloride-containing coated fine particles in (1) above and 54.6 g of lactose (450 mesh product, DMV Co.) were added to 40.8 g of water containing 1% by weight of Avicel RC-591NF to obtain a coated fine particle dispersion. 500 mg of this coated fine particle dispersion were filled into the holes of a concave mold having a diameter of 12 mm followed by freeze-drying (conditions: same as Example 1-(2)) to obtain orally fast disintegrating tablets of the present invention.

### Example 7

(1) A coating solution (prepared by dissolving 40 g of ethyl cellulose (Ethocel #10, available from Dow Chemical) and 40 g of stearic acid (available from Kao Corp.) in 920 g of ethanol) was sprayed onto 80 g of the diltiazem hydrochloride-containing core particles obtained in Example 6-(1) under fluidization in a Wurster fluidized bed coating device (GPCG-1, manufactured by Glatt) so that the coating ratio (ratio of coating layer to core particles) was 50% by weight to obtain coated fine particles. The resulting coated particles were sieved to obtain a fraction having a particle diameter of 75-250 µm as diltiazem hydrochloride-containing coated particles.
(2) 0.13 g of aspartame, 0.13 g of lemon flavor (available from Ogawa Flavors and Fragrances), 4.88 g of the diltiazem hydrochloride-containing coated fine particles in (1) above, and 11.38 g of lactose (200 mesh product, DMV Co.) were added to 8.5 g of water containing 1% by weight of Avicel RC-591NF to obtain a coated fine particle dispersion. 500 mg of this coated fine particle dispersion were filled into the holes of a concave mold having a diameter of 12 mm followed by freeze-drying (conditions: same as Example 1-(2)) to obtain orally fast disintegrating tablets of the present invention.

When the hardness of the resulting orally fast disintegrating tablets (6 tablets) was measured using a Tablet Tester (Model 6D, manufactured by Freund Industrial Co), it was found to be 10.0 N on average.

In addition, when the resulting orally fast disintegrating tablets were administered to three healthy adult males and the disintegration time in the oral cavity was measured, it was found to be 17 seconds on average. This demonstrates superior rapid disintegration in the oral cavity.

### Example 8

0.13 g of aspartame, 0.13 g of lemon flavor (available from Ogawa Flavors and Fragrances), 4.88 g of the diltiazem hydrochloride-containing coated fine particles obtained in Example 7-(1), 3.79 g of mannitol and 7.58 g of erythritol were added to 8.5 g of water containing 1% by weight of Avicel RC-591NF to obtain a coated fine particle dispersion. 500 mg of this coated fine particle dispersion were filled into the holes of a concave mold having a diameter of 12 mm followed by freeze-drying (conditions: same as Example 1-(2)) to obtain orally fast disintegrating tablets of the present invention.

When the hardness of the resulting orally fast disintegrating tablets (6 tablets) was measured using a Tablet Tester (Model 6D, manufactured by Freund Industrial Co), it was found to be 26.5 N on average.

### Example 9

175.7 g of acetaminophen and 70.3 g of hydroxypropyl cellulose were dissolved in 1054 g of ethanol, and this solution and 200 g of magnesium aluminate metasilicate (Neusilin NS2N, available from Fuji Chemical Industry) were mixed and stirred using a Shinagawa mixer followed by impregnation granulation. After drying the resulting particles for 16 hours at 45°C, they were sieved to obtain a fraction having a particle diameter of 75-150 µm as drug-containing core particles.

An ethyl cellulose solution (containing 3 parts by weight of Ethocel #10 (available from Dow Chemical), 63.05 parts by weight of ethanol and 33.95 parts by weight of water) was sprayed onto 80 g of the acetaminophen-containing core particles under fluidization in a Wurster fluidized bed coating device (GPCG-1, manufactured by Glatt) so that the coating ratio (ratio of coating layer to core particles) was 25% by weight to obtain coated fine particles. The resulting coated particles were sieved to obtain a fraction having a particle diameter of 75-250 µm as acetaminophen-containing coated particles.
(2) 0.13 g of aspartame, 0.13 g of lemon flavor (available from Ogawa Flavors and Fragrances), 4.88 g of the acetaminophen-containing coated fine particles in (1) above, and 11.38 g of lactose (200 mesh product, DMV Co.) were added to 8.5 g of water containing 1% by weight of Avicel RC-591NF to obtain a coated fine particle dispersion. 500 mg of this coated fine particle dispersion were filled into the holes of a concave mold having a diameter of 12 mm followed by freeze-drying (conditions: same as Example 1-(2)) to obtain orally fast disintegrating tablets of the present invention.

When the hardness of the resulting orally fast disintegrating tablets (6 tablets) was measured using a Tablet Tester (Model 6D, manufactured by Freund Industrial Co), it was found to be 15.8 N on average.

In addition, when the resulting orally fast disintegrating tablets were administered to three healthy adult males and the disintegration time in the oral cavity was measured, it was found to be 16 seconds on average. This demonstrates superior rapid disintegration in the oral cavity.

### Example 10

0.13 g of aspartame, 0.13 g of lemon flavor (available from Ogawa Flavors and Fragrances), 4.88 g of the acetaminophen-containing coated fine particles obtained in Example 9-(1), 3.79 g of mannitol and 7.58 g of erythritol were added to 8.5 g of water containing 1% by weight of Avicel RC-591NF to obtain a coated fine particle dispersion. 500 mg of this coated fine particle dispersion were filled into the holes of a concave mold having a diameter of 12 mm followed by freeze-drying (conditions: same as Example 1-(2)) to obtain orally fast disintegrating tablets of the present invention.

When the hardness of the resulting orally fast disintegrating tablets (6 tablets) was measured using a Tablet Tester (Model 6D, manufactured by Freund Industrial Co), it was found to be 16.7 N on average.

### Example 11

0.13 g of aspartame, 0.13 g of lemon flavor (available from Ogawa Flavors and Fragrances), 3.25 g of the acetaminophen-containing coated fine particles obtained in Example 9-(1), and 13 g of lactose (200 mesh product, DMV Co.) were added to 8.5 g of water containing 1% by weight of Avicel RC-591NF to obtain a coated fine particle dispersion. 500 mg of this coated fine particle dispersion were filled into the holes of a concave mold having a diameter of 12 mm followed by freeze-drying (conditions: same as Example 1-(2)) to obtain orally fast disintegrating tablets of the present invention.

When the hardness of the resulting orally fast disintegrating tablets (6 tablets) was measured using a Tablet Tester (Model 6D, manufactured by Freund Industrial Co), it was found to be 15.8 N on average.

In addition, when the resulting orally fast disintegrating tablets were administered to three healthy adult males and the disintegration time in the oral cavity was measured, it was found to be 11 seconds on average. This demonstrates superior rapid disintegration in the oral cavity.

### Example 12

0.13 g of aspartame, 0.13 g of lemon flavor (available from Ogawa Flavors and Fragrances), 3.75 g of the acetaminophen-containing coated fine particles obtained in Example 9-(1), and 11.25 g of lactose (200 mesh product, DMV Co.) were added to 9.75 g of water containing 1% by weight of Avicel RC-591NF to obtain a coated fine particle dispersion. 500 mg of this coated fine particle dispersion were filled into the holes of a concave mold having a diameter of 12 mm followed by freeze-drying (conditions: same as Example 1-(2)) to obtain orally fast disintegrating tablets of the present invention.

When the hardness of the resulting orally fast disintegrating tablets (6 tablets) was measured using a Tablet Tester (Model 6D, manufactured by Freund Industrial Co), it was found to be 12.0 N on average.

In addition, when the resulting orally fast disintegrating tablets were administered to three healthy adult males and the disintegration time in the oral cavity was measured, it was found to be 10 seconds on average. This demonstrates superior rapid disintegration in the oral cavity.

### Example 13

0.13 g of aspartame, 0.13 g of lemon flavor (available from Ogawa Flavors and Fragrances), 4.5 g of the acetaminophen-containing coated fine particles obtained in Example 9-(1), and 10.5 g of lactose (200 mesh product, DMV Co.) were added to 9.75 g of water containing 1% by weight of Avicel RC-591NF to obtain a coated fine particle dispersion. 500 mg of this coated fine particle dispersion were filled into the holes of a concave mold having a diameter of 12 mm followed by freeze-drying (conditions: same as Example 1-(2)) to obtain orally fast disintegrating tablets of the present invention.

When the hardness of the resulting orally fast disintegrating tablets (6 tablets) was measured using a Tablet Tester (Model 6D, manufactured by Freund Industrial Co), it was found to be 12.0 N on average.

In addition, when the resulting orally fast disintegrating tablets were administered to three healthy adult males and the disintegration time in the oral cavity was measured, it was found to be 12 seconds on average. This demonstrates superior rapid disintegration in the oral cavity.

### Example 14

0.13 g of aspartame, 0.13 g of lemon flavor (available from Ogawa Flavors and Fragrances), 5.25 g of the acetaminophen-containing coated fine particles obtained in Example 9-(1), and 9.75 g of lactose (200 mesh product, DMV Co.) were added to 9.75 g of water containing 1% by weight of Avicel RC-591NF to obtain a coated fine particle dispersion. 500 mg of this coated fine particle dispersion were filled into the holes of a concave mold having a diameter of 12 mm followed by freeze-drying (conditions: same as Example 1-(2)) to obtain orally fast disintegrating tablets of the present invention.

When the hardness of the resulting orally fast disintegrating tablets (6 tablets) was measured using a Tablet Tester (Model 6D, manufactured by Freund Industrial Co), it was found to be 13.8 N on average.

### Example 15

0.13 g of aspartame, 0.13 g of lemon flavor (available from Ogawa Flavors and Fragrances), 6 g of the acetaminophen-containing coated fine particles obtained in Example 9-(1), and 9 g of lactose (200 mesh product, DMV Co.) were added to 9.75 g of water containing 1% by weight of Avicel RC-591NF to obtain a coated fine particle dispersion. 500 mg of this coated fine particle dispersion were filled into the holes of a concave mold having a diameter of 12 mm followed by freeze-drying (conditions: same as Example 1-(2)) to obtain orally fast disintegrating tablets of the present invention.

When the hardness of the resulting orally fast disintegrating tablets (6 tablets) was measured using a Tablet Tester (Model 6D, manufactured by Freund Industrial Co), it was found to be 12.8 N on average.

### Example 16

0.13.g of aspartame, 0.13 g of lemon flavor (available from Ogawa Flavors and Fragrances), 6.75 g of the acetaminophen-containing coated fine particles obtained in Example 9-(1), and 8.25 g of lactose (200 mesh product, DMV Co.) were added to 9.75 g of water containing 1% by weight of Avicel RC-591NF to obtain a coated fine particle dispersion. 500 mg of this coated fine particle dispersion were filled into the holes of a concave mold having a diameter of 12 mm followed by freeze-drying (conditions: same as Example 1-(2)) to obtain orally fast disintegrating tablets of the present invention.

When the hardness of the resulting orally fast disintegrating tablets (6 tablets) was measured using a Tablet Tester (Model 6D, manufactured by Freund Industrial Co), it was found to be 12.7 N on average.

### Example 17

(1) 69.3 parts by weight of ecabet sodium pulverized with Sample Mill (manufactured by Fuji Paudal) and 29.7 parts by weight of partial α starch (PCS-PC10, available from Asahi Kasei) were stirred (600 rpm) using a high shear mixing granulator (New-Gra Machine NG-200, manufactured by Seishin Enterprises). 250 ml of a hydroxypropyl cellulose solution (prepared by dissolving 2 parts by weight of hydroxypropyl cellulose (HPC-SL, available from Nippon Soda) in a mixture of 49 parts by weight of ethanol and 49 parts by weight of water) were added thereto. The mixture was granulated for 24 minutes with stirring. After drying the resulting granulated particles for 16 hours at 45°C, they were sieved to obtain a fraction having a particle diameter of 75-150 µm as drug-containing core particles.
   A coating solution (prepared by dissolving 40 g of ethyl cellulose (Ethocel #10, available from Dow Chemical) and 10 g of stearic acid (available from Kao Corp.) in 950 g of ethanol) was sprayed onto 80 g of the ecabet sodium-containing core particles under fluidization in a Wurster fluidized bed coating device (GPCG-1, manufactured by Glatt) so that the coating ratio (ratio of coating layer to core particles) was 30% by weight to obtain coated fine particles. The resulting coated particles were sieved to obtain a fraction having a particle diameter of 75-250 µm as ecabet sodium-containing coated particles.
(2) 0.13 g of aspartame, 0.13 g of lemon flavor (available from Ogawa Flavors and Fragrances), 4.88 g of the ecabet sodium-containing coated fine particles obtained in (1) above, and 11.38 g of lactose (450 mesh product, DMV Co.) were added to 8.5 g of water containing 1% by weight of Avicel RC-591NF to obtain a coated fine particle dispersion. 500 mg of this coated fine particle dispersion were filled into the holes of a concave mold having a diameter of 12 mm followed by freeze-drying (conditions: same as Example 1-(2)) to obtain orally fast disintegrating tablets of the present invention.

When the hardness of the resulting orally fast disintegrating tablets (6 tablets) was measured using a Tablet Tester (Model 6D, manufactured by Freund Industrial Co), it was found to be 17.0 N on average.

### Example 18

(1) 40.0 parts by weight of diltiazem hydrochloride pulverized with Sample Mill (manufactured by Fuji Paudal), 40.0 parts by weight of mannitol and 20.0 parts by weight of microcrystalline cellulose (Avicel PH-M25, available from Asahi Kasei) were stirred (450 rpm) using a high shear mixing granulator (New-Gra Machine NG-350, manufactred by Seishin Enterprises). 435 ml of a hydroxypropyl cellulose solution (prepared by dissolving 2 parts by weight of hydroxypropyl cellulose (HPC-SL, available from Nippon Soda) in a mixture of 68 parts by weight of ethanol and 30 parts by weight of water) were added thereto. The mixture was granulated for 30 minutes with stirring. After drying the resulting granulated particles for 16 hours at 45°C, they were sieved to obtain a fraction having a particle diameter of 75-150 µm as drug-containing core particles.
   A coating solution (prepared by dissolving 40 g of ethyl cellulose (Ethocel #10, available from Dow Chemical) and 40 g of stearic acid (available from Kao Corp.) in 920 g of ethanol) was sprayed onto 80 g of the diltiazem hydrochloride-containing core particles under fluidization in a Wurster fluidized bed coating device (GPCG-1, manufactured by Glatt) so that the coating ratio (ratio of coating layer to core particles) was 60% by weight to obtain coated fine particles. The resulting coated particles were sieved to obtain a fraction having a particle diameter of 75-250 µm as diltiazem hydrochloride-containing coated particles.
(2) 0.8 g of aspartame, 0.8 g of strawberry flavor (available from Ogawa Flavors and Fragrances), 29.3 g of the diltiazem hydrochloride-containing coated fine particles in (1) above, and 68.3 g of lactose (450 mesh product, DMV Co.) were added to 51.0 g of water containing 2% by weight of Avicel RC-N81 to obtain a coated fine particle dispersion. 470 mg of this coated fine particle dispersion were filled into the holes of a concave mold having a diameter of 12 mm followed by freeze-drying (conditions: same as Example 1-(2)) to obtain orally fast disintegrating tablets of the present invention.

### Example 19

0.8 g of aspartame, 0.8 g of strawberry flavor (available from Ogawa Flavors and Fragrances), 29.3 g of the diltiazem hydrochloride-containing coated fine particles obtained in Example 18-(1), and 68.3 g of lactose (450 mesh product, DMV Co.) were added to 51.0 g of water containing 1% by weight of Avicel RC-N30 to obtain a coated fine particle dispersion. 470 mg of this coated fine particle dispersion were filled into the holes of a concave mold having a diameter of 12 mm followed by freeze-drying (conditions: same as Example 1-(2)) to obtain orally fast disintegrating tablets of the present invention.

### Example 20

(1) A coating solution (prepared by dissolving 40 g of ethyl cellulose (Ethocel #10, available from Dow Chemical) and 40 g of stearic acid (available from Kao Corp.) in 920 g of ethanol) was sprayed onto 80 g of the diltiazem hydrochloride-containing core particles obtained in Example 6-(1) under fluidization in a Wurster fluidized bed coating device (GPCG-1, manufactured by Glatt) so that the coating ratio (ratio of coating layer to core particles) was 40% by weight to obtain coated fine particles. The resulting coated particles were sieved to obtain a fraction having a particle diameter of 75-250 µm as diltiazem hydrochloride-containing coated particles.
(2) 3.0 g of aspartame, 3.0 g of strawberry flavor (available from Ogawa Flavors and Fragrances), 58.5 g of the diltiazem hydrochloride-containing coated fine particles in (1) above, and 136.5 g of lactose (450 mesh product, DMV Co.) were added to 102.0 g of water containing 1% by weight of Avicel RC-591NF to obtain a coated fine particle dispersion. 500 mg of this coated fine particle dispersion were filled into the holes of a concave mold having a diameter of 12 mm using a filling device (Mono-Dispenser, Model 4NDPL04G15, manufactured by Heishin) followed by freeze-drying (conditions: same as Example 1-(2)) to obtain orally fast disintegrating tablets of the present invention.

### Experimental Example 1

An aqueous dispersion containing 1% by weight of Avicel RC591NF was prepared by adding 99 parts by weight of water to 1 part by weight of Avicel RC-591NF, stirred for 1 hour with a magnetic stirrer and then allowed to stand for 16 hours. The viscosity thereof at 25°C was measured using a type B viscometer, it was found to be 17 mPa·s. Next, 1 part by weight of nifedipine particles (available from Wako Pure Chemical Industries, Lot No. KCR6473, average particle diameter: 72 µm, containing at a rate of 10% each of particles having a particle diameter of 36 µm or less and particles having a particle diameter of 147 µm or more) were added to 99 parts by weight of said dispersion and stirred for 2 hours to disperse. 50 ml thereof were poured into a Nessler tube (inner diameter: 22 mm) and then allowed to stand undisturbed. One ml aliquots were accurately sampled from the longitudinally central portion of the dispersion at 0 and 5 hours after the start of standing, followed by measurement of the concentrations of nifedipine particles. The concentrations of nifedipine particles in the samples were measured by (a) adding acetone to the sample to dissolve the nifedipine, (b) removing the Avicel RC-591NF by filtration, (c) diluting the filtrate with the second liquid of the disintegration test described in the 13th Revised Edition of the Japanese Pharmacopoeia, and (d) measuring the concentration of nifedipine in the diluted sample by measuring optical absorption (measured wavelength: 350 nm). The ratios (%) of the concentrations of nifedipine particles in the longitudinally central portion of the dispersion after allowing to stand for each amount of time are shown in Table 1 on the basis that the theoretical nifedipine particle concentration (amount of nifedipine particles/amount of dispersion) is 100.

Concomitantly, water and nifedipine particles separated completely within five hours in the case of dispersing nifedipine particles in water in the same manner as described above.

**Table 1**

| Standing time (hr) | Ratio (%) of nifedipine particle concentrations in center portion of dispersion after each standing time on the basis that the theoretical particle concentration is 100. |
|---|---|
| 0 | 100 |
| 5 | 94.7 |

As is clear from Table 1, the value after standing for 5 hours, namely the dispersion maintaining ratio, was 94.7%, thereby demonstrating that a satisfactory dispersed state is maintained for a long period of time when nifedipine particles are dispersed in water using Avicel RC-591NF (microcrystalline cellulose coated with carboxymethyl cellulose) as dispersant. As a result, in the process of the present invention, it was indicated that microcrystalline cellulose coated with sodium carboxymethyl cellulose (Avicel RC-591NF) can be used preferably as dispersant.

### Experimental Example 2

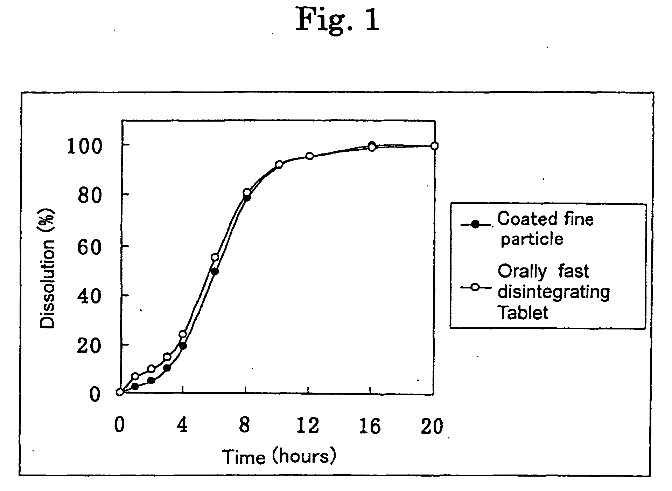

The dissolution characteristics of diltiazem hydrochloride were investigated for the coated fine particles obtained in Example 4(1) and the orally fast disintegrating tablets obtained in Example 4(2) by carrying out an dissolution test in compliance with the 13th Revised Edition of the Japanese Pharmacopoeia (test liquid: second liquid in the disintegration test). Those results are shown in Fig. 1.

As is clear from Fig. 1, the diltiazem hydrochloride dissolution characteristics of the coated fine particles and orally fast disintegrating tablets were nearly identical. on the basis of this finding, it was determined that the process of the present invention allows the production of an orally fast disintegrating preparation without impairing the function of the functional particles such as the coated particles.

### Experimental Example 3

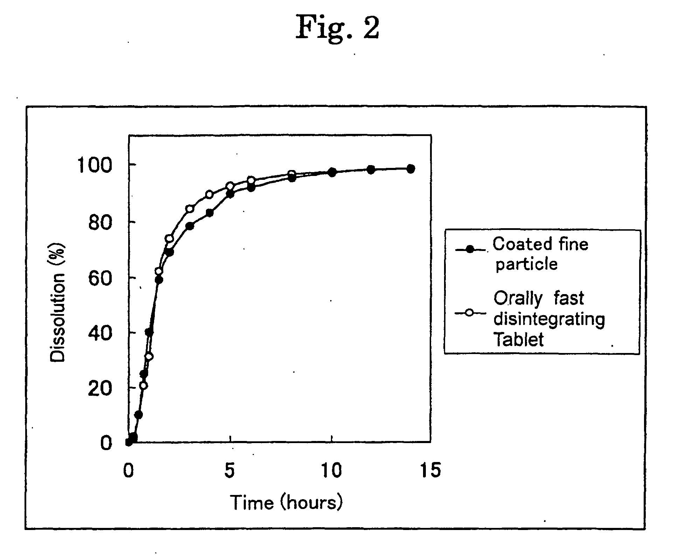

The dissolution characteristics of diltiazem hydrochloride were investigated for the coated fine particles obtained in Example 6(1) and the orally fast disintegrating tablets obtained in Example 6(2) by carrying out an dissolution test in compliance with the 13th Revised Edition of the Japanese Pharmacopoeia (test liquid: first liquid in the disintegration test). Those results are shown in Fig. 2.

As is clear from Fig. 2, the diltiazem hydrochloride dissolution characteristics of the coated fine particles and orally fast disintegrating tablets were nearly identical. On the basis of this finding, it was determined that the process of the present invention allows the production of an orally fast disintegrating preparation without impairing the function of the functional particles such as the coated particles.

### Experimental Example 4

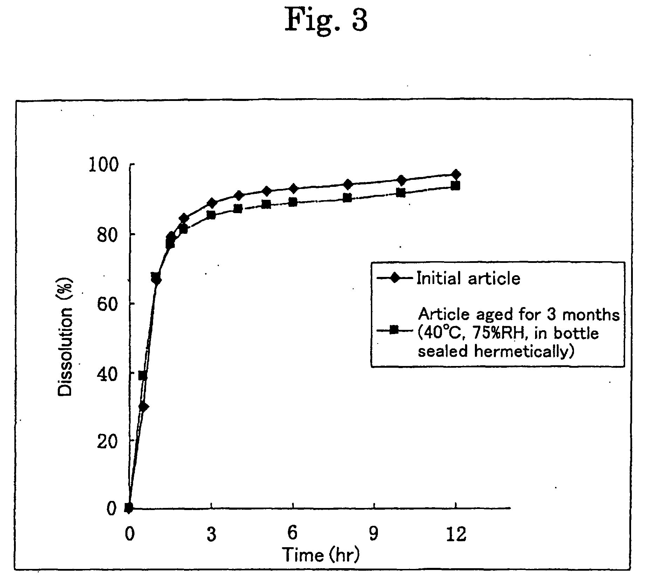

Storage stability was evaluated for the orally fast disintegrating tablets obtained in Example 6(2). Those results are shown in Fig. 3 (dissolution test method: same as Experimental Example 3).

As is clear from Fig. 3, it was determined that the process of the present invention allows the obtaining of an orally fast disintegrating preparation, which has a high level of storage stability.

### Experimental Example 5

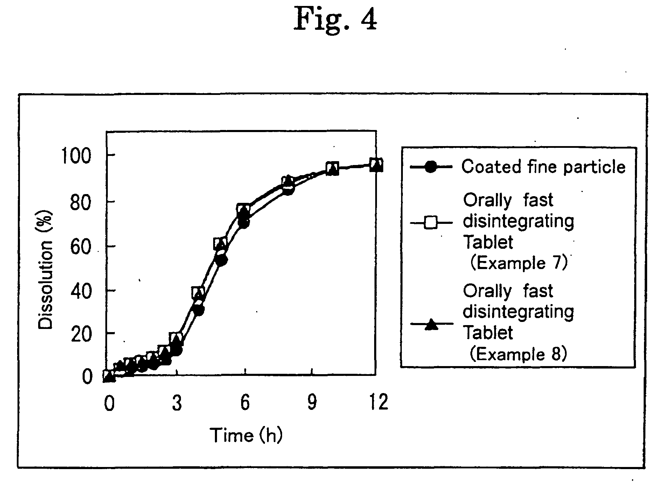

The dissolution characteristics of diltiazem hydrochloride were investigated for the coated fine particles obtained in Example 7(1) and the orally fast disintegrating tablets obtained in Example 7(2) and Example 8 by carrying out an dissolution test in compliance with the 13th Revised Edition of the Japanese Pharmacopoeia (test liquid: second liquid in the disintegration test). Those results are shown in Fig. 4.

As is clear from Fig. 4, the diltiazem hydrochloride dissolution characteristics of the coated fine particles and orally fast disintegrating tablets were nearly identical. On the basis of this finding, it was determined that the process of the present invention allows the production of an orally fast disintegrating preparation.without impairing the function of the functional particles such as the coated particles.

### Experimental Example 6

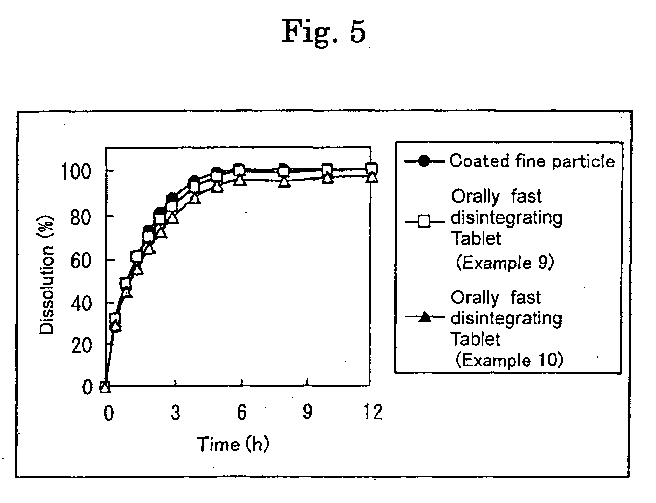

The dissolution characteristics of acetaminophen were investigated for the coated fine particles obtained in Example 9(1) and the orally fast disintegrating tablets obtained in Example 9(2) and Example 10 by carrying out an dissolution test in compliance with the 13th Revised Edition of the Japanese Pharmacopoeia (test liquid: second liquid in the disintegration test). Those results are shown in Fig. 5.

As is clear from Fig. 5, the acetaminophen dissolution characteristics of the coated fine particles and orally fast disintegrating tablets were nearly identical. On the basis of this finding, it was determined that the process of the present invention allows the production of an orally fast disintegrating preparation without impairing the function of the functional particles such as the coated particles.

### Experimental Example 7

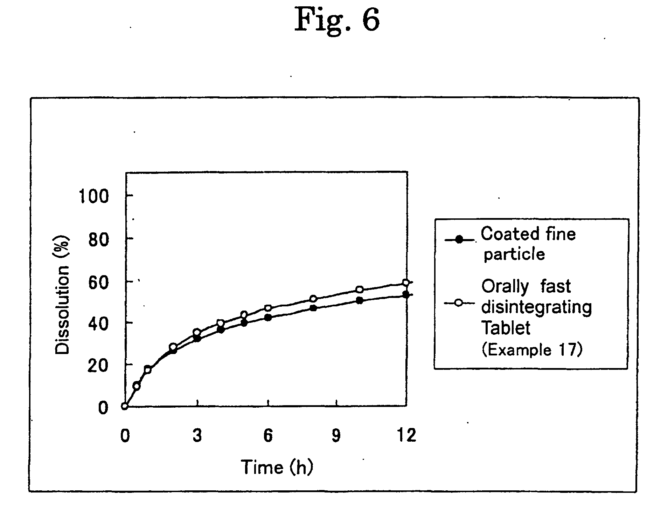

The dissolution characteristics of ecabet sodium were investigated for the coated fine particles obtained in Example 17(1) and the orally fast disintegrating tablets obtained in Example 17(2) by carrying out an dissolution'test in compliance with the 13th Revised Edition of the Japanese Pharmacopoeia (test liquid: second liquid in the disintegration test). Those results are shown in Fig. 6.

As is clear from Fig. 6, the ecabet sodium dissolution characteristics of the coated fine particles and orally fast disintegrating tablets were nearly identical. On the basis of this finding, it was determined that the process of the present invention allows the production of an orally fast disintegrating preparation without impairing the function of the functional particles such as the coated particles.

### Experimental Example 8

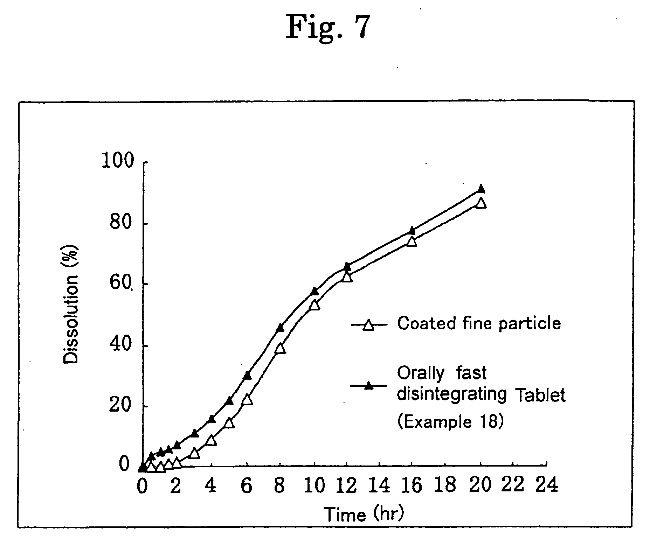

The dissolution characteristics of diltiazem hydrochloride were investigated for the coated fine particles obtained in Example 18(1) and the orally fast disintegrating tablets obtained in Example 18(2) by carrying out an dissolution test in compliance with the 13th Revised Edition of the Japanese Pharmacopoeia (test liquid: second liquid in the disintegration test). Those results are shown in Fig. 7.

As is clear from Fig. 7, the diltiazem hydrochloride dissolution characteristics of the coated fine particles and orally fast disintegrating tablets were nearly identical. On the basis of this finding, it was determined that the process of the present invention allows the production of an orally fast disintegrating preparation without impairing the function of the functional particles such as the coated particles.

### Experimental Example 9

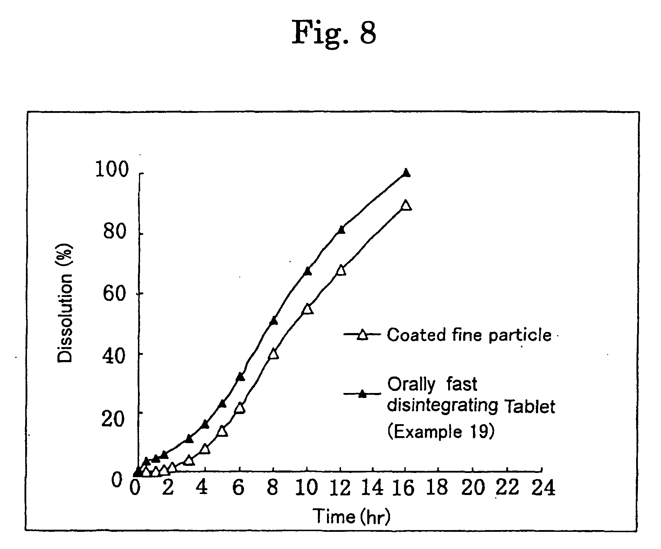

The dissolution characteristics of diltiazem hydrochloride were investigated for the coated fine particles obtained in Example 18(1) and the orally fast disintegrating tablets obtained in Example 19(2) by carrying out an dissolution test in compliance with the 13th Revised Edition of the Japanese Pharmacopoeia (test liquid: second liquid in the disintegration test). Those results are shown in Fig. 8.

As is clear from Fig. 8, the diltiazem hydrochloride dissolution characteristics of the coated fine particles and orally fast disintegrating tablets were nearly identical. On the basis of this finding, it was determined that the process of the present invention allows the production of an orally fast disintegrating preparation without impairing the function of the functional particles such as the coated particles.

### Experimental Example 10

Samples were taken from the orally fast disintegrating tablets obtained in Example 20(2) at the early stage of filling (up to 100 tablets from the start of filling), at the intermediate stage of filling (200-400 tablets after the start of filling) and at the late stage of filling (tablets filled after allowing to stand for 3 hours in the filling device). The weight uniformity (n = 20) and the content uniformity (n = 10) of the tablets were investigated for each of the samples. Those results are shown in Table 2.

**Table 2**

| Weight Uniformity and Content Uniformity | | | | |
|---|---|---|---|---|
| Measured Parameters | | Sampling Time | | |
| | | Early stage of filling (Up to 100 tablets) | Intermediate stage of filling (200-400 tablets) | Late stage of filling (filling after standing for 3 hours) |
| Weight | Avg.(mg/tablet) | 335.1 | 334.1 | 334.1 |
| Uniformity | σn-1 | 1.6 | 1.2 | 0.9 |
| (n = 20) | C.V. value (%) | 0.5 | 0.4 | 0.3 |
| Content | Avg.(mg/tablet) | 37.1 | 37.6 | 38.4 |
| Uniformity | σn-1 | 0.5 | 0.3 | 0.9 |
| (n = 10) | C.V. value (%) | 1.3 | 0.7 | 2.4 |

As is clear from Table 2, there was no weight or content segregation caused by phase separation and so forth which might have been observed at any stage of filling. On the basis of this finding, the process of the present invention was found to allow the filling liquid to have superior dispersion, allow functional particles such as coated particles to be uniformly dispersed in the filling liquid, and allow uniform filling.

### Reference Example 1

A suspension was obtained by adding 0.2 part by weight of aspartame, 33.4 parts by weight of mannitol and 33 parts by weight of erythritol to 33.4 parts by weight of water containing 0.5% by weight of Avicel RC-591NF. 500 mg of this suspension were filled into the holes of a concave mold having a diameter of 10 mm followed by freeze-drying to obtain orally fast disintegrating tablets.

### Reference Example 2

A suspension was obtained by adding 0.2 part by weight of aspartame, 33.4 parts by weight of mannitol and 33 parts by weight of lactose to 33.4 parts by weight of water containing 0.5% by weight of Avicel RC-591NF. 500 mg of this suspension were filled into the holes of a concave mold having a diameter of 10 mm followed by freeze-drying to obtain orally fast disintegrating tablets.

### Reference Example 3

A suspension was obtained by adding 0.2 part by weight of aspartame, 33.4 parts by weight of lactose and 33 parts by weight of erythritol to 33.4 parts by weight of water containing 0.5% by weight of Avicel RC-591NF. 500 mg of this suspension were filled into the holes of a concave mold having a diameter of 10 mm followed by freeze-drying to obtain orally fast disintegrating tablets.

### Reference Example 4

A suspension was obtained by adding 0.2 part by weight of aspartame and 66.4 parts by weight of lactose to 33.4 parts by weight of water containing 0.5% by weight of Avicel RC-591NF. 500 mg of this suspension were filled into the holes of a concave mold having a diameter of 10 mm followed by freeze-drying to obtain orally fast disintegrating tablets.

### Reference Example 5

A suspension was obtained by adding 0.2 part by weight of aspartame, 33.4 parts by weight of mannitol and 33 parts by weight of maltose to 33.4 parts by weight of water containing 0.5% by weight of Avicel RC-591NF. 500 mg of this suspension were filled into the holes of a concave mold having a diameter of 10 mm followed by freeze-drying to obtain orally fast disintegrating tablets.

### Industrial Applicability

An orally fast disintegrating preparation containing functional particles produced according to the process of the present invention is characterized in that, the functional particles are not subjected to damage by compression molding, there are no variations in the content of functional particles among preparations, the oral feeling at dosing is satisfactory, the preparation is inert to bacterial growth and a drying step is easy.

## Claims

1. A process for producing an orally fast disintegrating preparation containing functional particles, which comprises:
filling in a mold an aqueous dispersion containing (a) a dispersant showing a dispersion maintaining ratio of 75% or more and a viscosity of 100 mPa^{·}s or less at 25°C in case of being contained homogeneously in water at 1% by weight, (b) a water-soluble saccharide, and (c) functional particles; and then
removing water.

2. The process according to claim 1, wherein the dispersant is a complex composed of fine particles of a water-insoluble substance and a water-soluble substance.

3. The process according to claim 2, wherein the dispersant is a complex obtainable by drying an aqueous dispersion containing fine particles of a water-insoluble substance and a water-soluble substance.

4. The process according to claim 2 or 3, wherein the water-insoluble substance is a fibrous substance.

5. The process according to claim 4, wherein the fine particles of the water-insoluble substance are microcrystalline cellulose.

6. The process according to any of claims 2-5, wherein an average particle diameter of the fine particles of the water-insoluble substance is 30 µm or less.

7. The process according to any of claims 2-6, wherein the water-soluble substance is one or more than one selected from locust bean gum, guar gum, tamarind gum, quince seed gum, karaya gum, gum Arabic, tragacanth gum, gaty gum, arabinogalactan, agar, carrageenan, alginic acid and its salts, furcellaran, pectin, marmelo, xanthane gum, cardran, pullulan, dextran, gellan gum, gelatin, sodium carboxymethyl cellulose, sodium polyacrylate, sodium chondroitin sulfate, sodium glycolate starch, starch hydrolysates, dextrins, glucose, fructose, xylose, sorbose, sucrose, lactose, maltose, isomerized sugar, coupling sugar, palatinose, neo sugar, reduced starch saccharified syrup, lactulose, polydextrose, fructooligosaccharide, galactooligosaccharide, xylitol, mannitol, maltitol and sorbitol.

8. The process according to claim 1, wherein the dispersant is microcrystalline cellulose coated with one or more than one selected from sodium carboxymethyl cellulose, a mixture of karaya gum and dextrin, and a mixture of xanthane gum and dextrin.

9. The process according to claim 8, wherein the dispersant is microcrystalline cellulose coated with sodium carboxymethyl cellulose.

10. The process according to any of claims 1-9, wherein the water-soluble saccharide is one or more than one selected from glucose, fructose, sucrose, lactose, maltose, mannitol, xylitol, sorbitol, trehalose and erythritol.

11. The process according to claim 10, wherein the water-soluble saccharide is one or more than one selected from lactose, maltose, mannitol and erythritol.

12. The process according to any of claims 1-11, wherein an average particle diameter of the functional particles is 50-200 µm, and a ratio of the functional particles having a particle diameter of 250 µm or more is 10% or less of a total amount of the functional particles.

13. The process according to any of claims 1-12, wherein the functional particles are coated particles.

14. The process according to claim 1, wherein a content of the dispersant in the aqueous dispersion is 0.05-2.0% by weight.

15. The production process according to claim 14, wherein a blended amount of the water-soluble saccharide in the aqueous dispersion is 30-1000 parts by weight relative to 1 part by weight of the dispersant, and a blended amount of the functional particles is 1-500 parts by weight relative to 1 part by weight of the dispersant.

16. The process according to claim 14 or 15, wherein the water-soluble saccharide is not completely dissolved in water in the aqueous dispersion.

17. The process according to any of claims 1-16, wherein the water is removed from the aqueous dispersion containing the functional particles by freeze-drying.

18. An orally fast disintegrating preparation containing functional particles, which comprises (a) a dispersant showing a dispersion maintaining ratio of 75% or more and a viscosity of 100 mPa^{·}s or less at 25°C in case of being contained homogeneously in water at 1% by weight, (b) a water-soluble saccharide, and (c) functional particles.

19. An orally fast disintegrating preparation containing functional particles, which is obtainable by filling in a mold an aqueous dispersion containing (a) a dispersant showing a dispersion maintaining ratio of 75% or more and a viscosity of 100 mPa^{·}s or less at 25°C in case of being contained homogeneously in water at 1% by weight, (b) a water-soluble saccharide, and (c) functional particles; and then
removing water.

20. The orally fast disintegrating preparation containing functional particles according to claim 18 or 19, wherein a content of the dispersion in the preparation is 0.07-4% by weight.

21. The orally fast disintegrating preparation containing functional particles according to any of claims 18-20, wherein a blended amount of the water-soluble saccharide is 30-1000 parts by weight relative to 1 part by weight of the dispersant, and a blended amount of the functional particles is 1-500 parts by weight relative to 1 part by weight of the dispersant, in the preparation.

22. The orally fast disintegrating preparation containing functional particles according to any of claims 18-21, wherein a hardness is 10 N or more, and a disintegration time in the oral cavity is 60 seconds or shorter.
